# EUROPEAN PATENT SPECIFICATION

(11) **EP 3 441 066 B1**
(45) Date of publication and mention of the grant of the patent: **29.11.2023**
(21) Application number: 16897732.0
(22) Date of filing: 14.10.2016
(51) Int. Cl.: A61K 31/277, A61K 31/166, A61K 31/519, A61P 25/16

(54) **MAPK INHIBITORS FOR THE TREATMENT OF PARKINSON'S DISEASE**
MAPK-INHIBITOREN FÜR DIE BEHANDLUNG DER PARKINSON-KRANKHEIT
INHIBITEURS DE MAPK POUR LE TRAITEMENT DE LA MALADIE DE PARKINSON

(30) Priority: 06.04.2016 CN 201610208614; 06.04.2016 CN 201610208591
(43) Date of publication of application: 13.02.2019
(73) Proprietor: Fuzhou University, Fuzhou, Fujian 350108 (CN)
(72) Inventor: YANG, Yufeng, Fuzhou Fujian 350108 (CN)
(74) Representative: Sagittarius IP
(86) International application number: PCT/CN2016/102154
(87) International publication number: WO 2017/173800

(56) References cited:
- WO-A1-2013/093137
- CN-A- 104 918 937
- CN-A- 105 796 544
- CN-A- 105 816 461
- US-A1- 2013 303 788
- WENYA WANG ET AL: "SP600125, a new JNK inhibitor, protects dopaminergic neurons in the MPTP model of Parkinson's disease", NEUROSCIENCE RESEARCH., vol. 48, no. 2, February 2004 (2004-02), pages 195-202, XP055629652, IR ISSN: 0168-0102, DOI: 10.1016/j.neures.2003.10.012
- SE-EUN PARK ET AL: "Rutin from Dendropanax morbifera Leveille Protects Human Dopaminergic Cells Against Rotenone Induced Cell Injury Through Inhibiting JNK and p38 MAPK Signaling", NEUROCHEMICAL RESEARCH., vol. 39, no. 4, 19 February 2014 (2014-02-19), pages 707-718, XP055629640, US ISSN: 0364-3190, DOI: 10.1007/s11064-014-1259-5
- LORRAINE V KALIA ET AL: "Parkinson's disease", LANCET, vol. 386, no. 9996, 20 April 2015 (2015-04-20), pages 896-912, XP055491886, AMSTERDAM, NL ISSN: 0140-6736, DOI: 10.1016/S0140-6736(14)61393-3
- ZHANG, ZUOFENG et al.: "Effect of phosphorylated-ERK1/2 on NF-KB p65 expression in substantia nigra of mice with MPTP-induced Parkinson's disease", Academic Journal of Second Military Medical University, vol. 32, no. 11, 30 November 2011 (2011-11-30), pages 1176-1180, XP009511812, ISSN: 0258-879X
- Kimberly Burkhard ET AL: "Use of Inhibitors in the Study of MAP Kinases", Methods in molecular biology, vol. 661, 1 January 2010 (2010-01-01), pages 107-122, XP055560492, US ISSN: 1064-3745, DOI: 10.1007/978-1-60761-795-2_6 ISBN: 978-1-60761-961-1
- VALHMU W B ET AL: "DIFFERENTIAL REGULATION OF AGGRECAN GENE EXPRESSION BY MEK INHIBITORS: A NOVEL MECHANISM?", TRANSACTIONS OF THE ANNUAL MEETING - ORTOPAEDIC RESEARCH SOCIETY, 1 January 2002 (2002-01-01), page 0058,

## Description

### Cross reference

The present application claims the priorities of Chinese patent application 201610208614.8 submitted on April 6, 2016 and Chinese patent applications 201610208591.0 submitted on April 6, 2016.

### Technical field

The present invention belongs to the technical field of medicine and specifically the present invention relates to a MAPK signaling pathway inhibitor selected from the group consisting of PD0325901 and trametinib for use in the treatment of Parkinson's Disease.

### Background art

Dopaminergic (dopamine, DA) neurons are closely related to Parkinson's Disease (PD). People first recognized PD (also known as "tremor palsy") and then in the continuous exploration of its pathogenesis, began to understand the dopaminergic neurons and established their central role in the pathogenesis of PD. PD is a common degenerative disease of the central nervous system, which mainly affects middle-aged and elderly people. The majority of the patients come down with this disease after 50 years of age, but in recent years, younger people have been found to suffer from this disease. The main symptoms of the patients with PD are slow movements, tremors that cannot be controlled by the hands, feet or other parts of the body, body stiffness and balance disorders, leading to a failure to take care of themselves. According to statistics, the prevalence of PD in populations over the age of 60 in China is about 1.7%, there are more than 1 million patients in total, and there is an increase of at least 100,000 new patients each year. The degeneration and death of dopaminergic neurons in the substantia nigra of the midbrain and the significant reduction in the DA content of the striatum leading to incapability to meet the normal needs of the human bodies are one of the main causes of PD.

At present, PD is mainly treated by drugs or drugs assisted by surgeries. There are principally three commonly-used drugs: the first is a dopamine formulation such as Levodopa, which can provide a direct supplement of dopamine insufficiently secreted in the brain; the second is a drug that inhibits the degradation of dopamine, which can reduce the degradation of dopamine and relatively increase the dopamine content in the brain; the third is a dopamine receptor agonist that can enhance the absorption and utilization of dopamine in the brain. However, none of them can delay the degeneration of dopaminergic neurons and thus they cannot fundamentally achieve a reversing therapeutic effect.

ZHANG et al. (Academic Journal of Second Military Medical University, vol. 32, no. 11, 2011, pages 1176-1180) discloses that U0126 significantly inhibited the activation of ERK1/2 in the substantia nigra when administered in the MPTP-induced PD mouse model, and the loss of dopamine neurons was reduced. WO2013/093137 shows that the use of a MEK inhibitor named PD098059 is a memory enhancer and mentions the use of related agents for enhancing memory in PD and other diseases.

Therefore, there is still a need for drugs that can delay the degeneration of dopaminergic neurons and fundamentally treat PD.

### Summary of the invention

It is an object of the present invention to provide a medicament capable of delaying the degeneration of dopaminergic neurons, particularly a medicament capable of treating PD, which is achieved by the Applicant in discovering a new use of the MAPK signaling pathway inhibitor by inventive effort.

Therefore, described herein is an agent for use in a method of delaying degeneration of dopaminergic neurons in 4 disease animal models in need thereof, the method comprising administering to the disease models a therapeutically effective amount of an MAPK signaling pathway inhibitor.

The invention provides a MEK inhibitor selected from the group consisting of PD0325901 and trametinib for use in the treatment of Parkinson's Disease.

Described herein is an MAPK signaling pathway inhibitor for use in delaying degeneration of dopaminergic neurons.

Described herein is an MAPK signaling pathway inhibitor for use in treating PD animal models.

According to one aspect of the above embodiments of the present invention, the MAPK signaling pathway inhibitor comprises a MEK inhibitor and an ERK antagonist.

According to the present invention, the MAPK signaling pathway inhibitor is selected from PD0325901 and trametinib.

According to a particularly preferred embodiment of the present invention, the MAPK signaling pathway inhibitor is trametinib.

A significant advantage described herein is that an MAPK signaling pathway inhibitor is used in delaying the degeneration of dopaminergic neurons. The resulting product can effectively delay the degenerative death of dopaminergic neurons and fundamentally achieve the effect of treating PD animal models. Moreover, the medicament does not need to be injected and the therapeutic effect can be achieved only by oral administration; the medicament has a better therapeutic effect on PD caused by various causes and is of great significance for the treatment and cure of PD.

### Brief description of the drawings

Figure 1 is a schematic diagram of the morphology of dopaminergic neurons in the local brain regions of the fruit flies treated with U0126.
Figure 2 is a statistical diagram of the quantity of dopaminergic neurons in the local brain regions of the fruit flies treated with U0126. NS indicates no statistically significant difference; ** indicates a statistically significant difference according to student t-test, p < 0.05.
Figure 3 is a schematic diagram of the morphology of dopaminergic neurons in the local brain regions of the fruit flies treated with PD0325901.
Figure 4 is a statistical diagram of the quantity of dopaminergic neurons in the local brain regions of the fruit flies treated with PD0325901. NS indicates no statistically significant difference; ** indicates a statistically significant difference according to student t-test, p < 0.05.
Figure 5 is a schematic diagram of the morphology of dopaminergic neurons in the local brain regions of the fruit flies treated with trametinib.
Figure 6 is a statistical diagram of the quantity of dopaminergic neurons in the local brain regions of the fruit flies treated with trametinib. NS indicates no statistically significant difference; ** indicates a statistically significant difference according to student t-test, p < 0.05.

### Specific embodiments

In order to make the contents of the present invention easier to understand, the technical solutions described in the present invention are further described below in conjunction with specific embodiments, but the present invention is not only limited thereto.

### Examples

Example 1: Effect of U0126 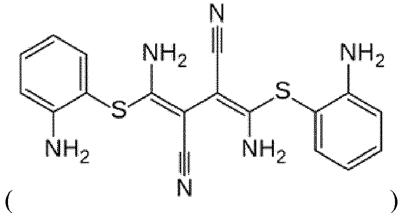 on dopaminergic neurons in local brain regions (reference)

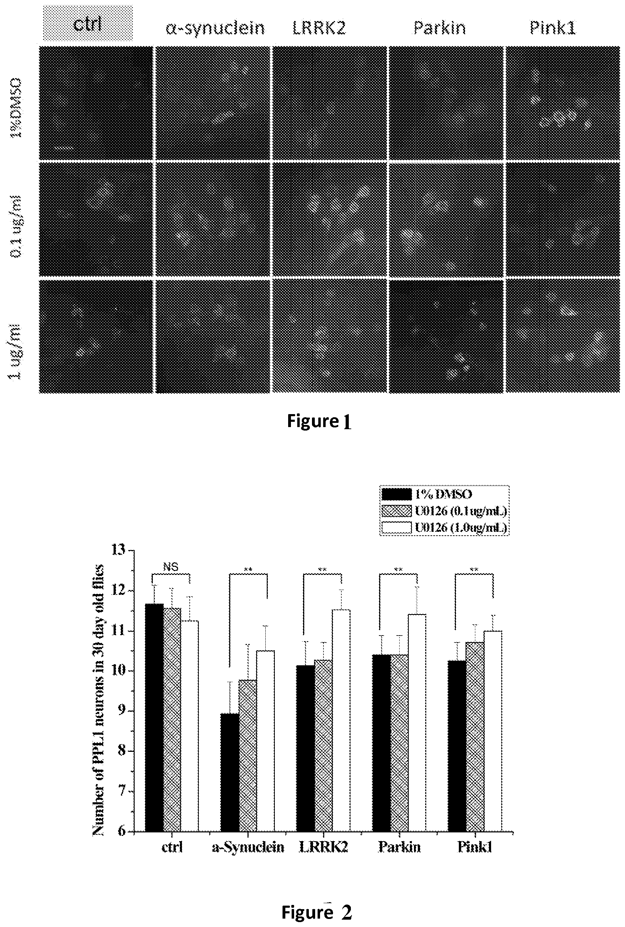

450 fruit flies of *Drosophila melanogaster* (purchased from Bloomington *Drosophila* Stock Center, USA) were divided into 4 PD disease model groups (as for "model groups", each group has the same genotype and different groups have different genotypes; see the following) and 1 normal control group (Ctrl, non-PD type). Animal models of PD caused by alpha-synuclein transgene overexpression, Lrrk2 transgene overexpression, Parkin gene mutation and Pink1 gene mutation were established in the four model groups. Each group includes 90 fruit flies and each group was further equally divided into three groups, drug-feeding groups and non-drug-feeding control group, for testing. Starting from imago at the age of 5 days, the drug-feeding group was continuously fed with 0.1 µg/mL, 1 µg/mL U0126 (U0126 is dissolved in 1% DMSO) for 30 days and the non-drug-feeding control group was fed with 1% DMSO as a solvent control. After 30 days of drug or control solvent feeding, the brains of fruit flies were dissected and the dopamine neurons were labeled by the whole-tissue immunostaining of the brains of the fruit flies with an anti-dopamine hydroxylase antibody. The morphology and quantity of the dopamine neurons in the PPL regions in the brains of the fruit flies were observed microscopically to determine the effect of U0126 on the dopaminergic neurons in the local brain regions. The results thereof were shown in Figures 1 and 2.

Figure 1 is a schematic diagram of the morphology of dopaminergic neurons in the local brain region after treatment with U0126, wherein the fluorescent part is the cell body profile of the dopaminergic neurons, through which the state and quantity of the dopaminergic neurons can be observed.

Figure 2 is a statistical diagram of the quantity of the dopaminergic neurons in the PPL brain region after treatment with U0126.

As can be seen from Figures 1 and 2, 1 µg/mL U0126 can effectively reverse the degenerative death of the dopaminergic neurons in the pathological model animals with PD caused by different genetic abnormalities and even restore it to normal.

Example 2: Effect of PD0325901 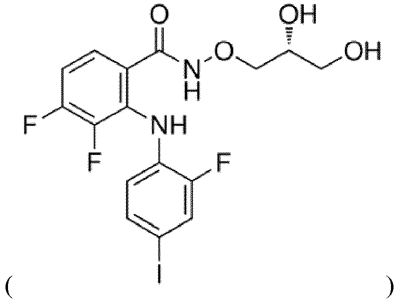 on dopaminergic neurons in local brain regions

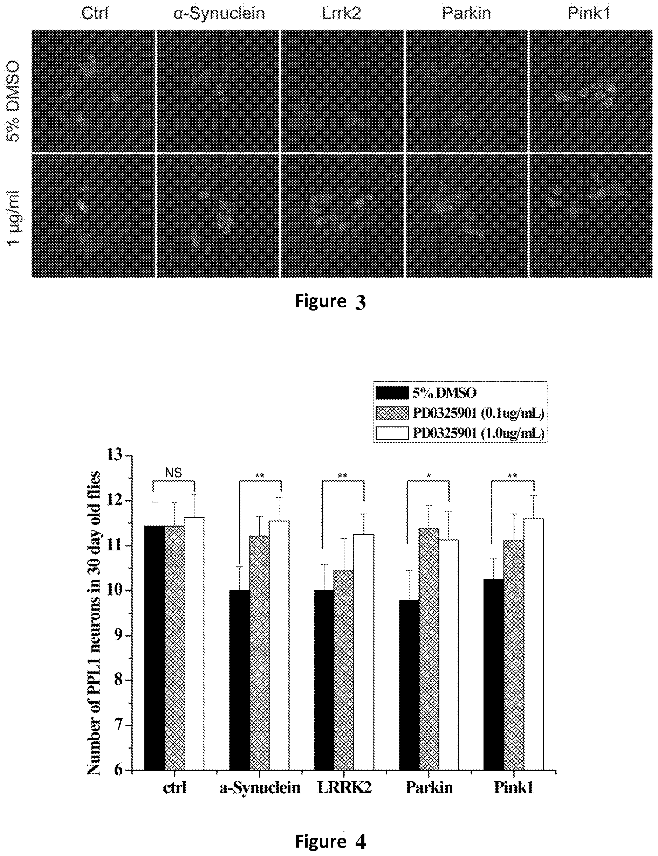

450 fruit flies of *Drosophila melanogaster* (purchased from Bloomington *Drosophila* Stock Center, USA) were divided into 4 PD disease model groups (as for "model groups", each group has the same genotype and different groups have different genotypes; see the following) and 1 normal control group (Ctrl, non-PD type). Animal models of PD caused by alpha-synuclein transgene overexpression, Lrrk2 transgene overexpression, Parkin gene mutation and Pink1 gene mutation were established in the four model groups. Each group includes 90 fruit flies and each group was further equally divided into 2 drug-feeding groups and 1 non-drug-feeding control group for testing. Starting from imago at the age of 5 days, the drug-feeding groups were respectively continuously fed with 0.1 µg/mL, 1 µg/mL PD0325901 (PD0325901 is dissolved in 1% DMSO) for 30 days and the normal control group was fed with 5% DMSO as a solvent control. After 30 days of drug or control solvent feeding, the brains of fruit flies were dissected and the dopamine neurons were labeled by the whole-tissue immunostaining of the brains of the fruit flies with an anti-dopamine hydroxylase antibody. The morphology and quantity of the dopamine neurons in the PPL regions in the brains of the fruit flies were observed microscopically to determine the effect of PD0325901 on the dopaminergic neurons in the local brain regions of the fruit flies. The results thereof were shown in Figures 3 and 4.

Figure 3 is a schematic diagram of the morpohology of dopaminergic neurons in the local brain region after treatment with PD0325901, wherein the fluorescent part is the cell body profile of the dopaminergic neurons, through which the morphology and quantity of the dopaminergic neurons can be observed. Figure 4 is a statistical diagram of the quantity of the dopaminergic neurons in the PPL brain region after treatment with PD0325901. As can be seen from Figures 3 and 4, 1 µg/mL PD0325901 can effectively reverse the degenerative loss of the dopaminergic neurons in the pathological model animals with PD caused by different genetic abnormalities and even restore it to normal.

Example 3: Effect of trametinib (the chemical name is (N-(3-{3-cyanopropyl-5-[(2-fluoro-4-iodophenyl)amino]-6,8-dimethyl-2,4,7-trioxo-3,4,6,7-tetrahyd ropyrido[4,3-d]pyrimidin-1(2H)yl}phenyl)acetamide); the molecular formula is C₂₆H₂₃FIN₅O₄; the structural formula is 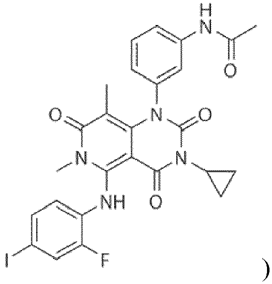 on dopaminergic neurons in local brain regions

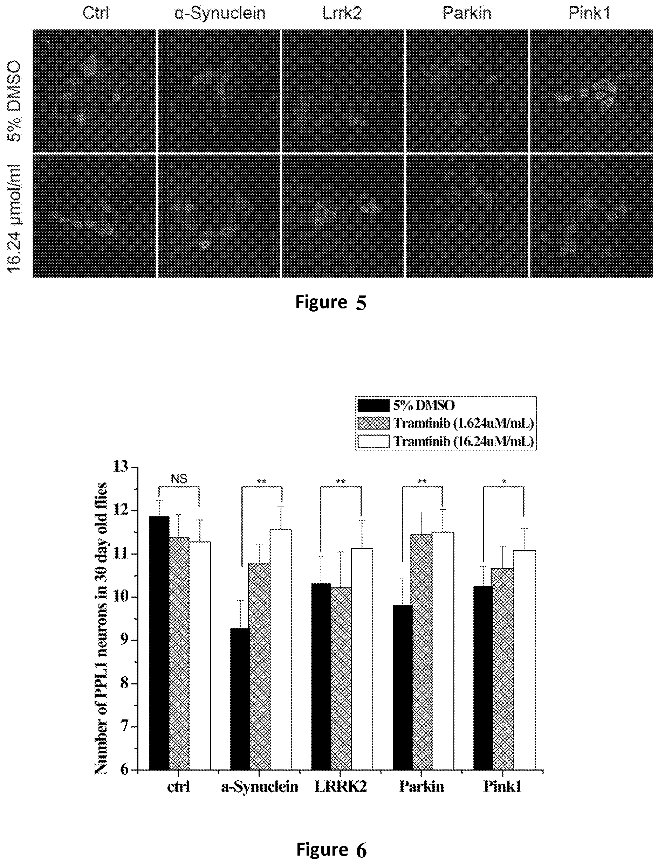

As experimental objects, 450 fruit flies of *Drosophila melanogaster* (purchased from Bloomington *Drosophila* Stock Center, USA) were divided into 4 PD disease model groups (as for "model groups", each group has the same genotype and different groups have different genotypes; see the following) and 1 normal control group (Ctrl, non-PD type). Animal models of PD caused by alpha-synuclein transgene overexpression, Lrrk2 transgene overexpression, Parkin gene mutation and Pink1 gene mutation were established in the four model groups. Each group includes 90 fruit flies and each group was further equally divided into 2 drug-feeding groups and 1 non-drug-feeding control group for testing. Starting from imago at the age of 5 days, the drug-feeding groups were respectively continuously fed with 1.624 µM/mL, 16.24 µM/mL trametinib (trametinib is dissolved in 1% DMSO) for 30 days and the non-drug-feeding control group was fed with 5% DMSO as a solvent control. After 30 days of drug or control solvent feeding, the brains of fruit flies were dissected and the dopamine neurons were labeled by the whole-tissue immunostaining of the brains of the fruit flies with an anti-dopamine hydroxylase antibody. The morphology and quantity of the dopamine neurons in the PPL regions in the brains of the fruit flies were observed microscopically to determine the effect of trametinib on the dopaminergic neurons in the local brain regions of the fruit flies. The results thereof were shown in Figures 5 and 6.

Figure 5 is a schematic diagram of the morphology of dopaminergic neurons in the local brain region after treatment with trametinib, wherein the fluorescent part is the cell body profile of the dopaminergic neurons, through which the state and quantity of the dopaminergic neurons can be observed. Figure 6 is a statistical diagram of the quantity of the dopaminergic neurons in the PPL brain region after treatment with trametinib. As can be seen from Figures 5 and 6, 16.24 µM/mL trametinib can effectively reverse the degenerative death of the dopaminergic neurons in the pathological model animals with PD caused by different genetic abnormalities and even restore it to normal.

Therefore, by providingMAPK signaling pathway inhibitors for use in treating PD, the health of the dopaminergic neurons can be maintained and the patients with PD are expected to be treated fundamentally.

## Claims

1. A MAPK signaling pathway inhibitor selected from the group consisting of PD0325901 and trametinib for use in the treatment of Parkinson's Disease.

2. The MAPK signaling pathway MEK inhibitor for use according to claim 1, which is trametinib.

## Patentansprüche

1. MAPK-Signalweg-Inhibitor, ausgewählt aus der Gruppe bestehend aus PD0325901 und Trametinib zur Verwendung bei der Behandlung der Parkinson-Krankheit.

2. MAPK-Signalweg-Inhibitor zur Verwendung nach Anspruch 1, der Trametinib ist.

## Revendications

1. Inhibiteur de la voie de signalisation MAPK sélectionné dans le groupe constitué de PD0325901 et du trametinib destiné à être utilisé dans le traitement de la maladie de Parkinson.

2. Inhibiteur de la voie de signalisation MAPK destiné à être utilisé selon la revendication 1, qui est le trametinib.
